# EUROPÄISCHE PATENTSCHRIFT

(11) **EP 0 698 394 B1**
(45) Veröffentlichungstag und Bekanntmachung des Hinweises auf die Patenterteilung: **09.06.1999**
(21) Anmeldenummer: 95112625.9
(22) Anmeldetag: 11.08.1995
(51) Int. Cl.: A61L 9/20, B01D 53/66, A61L 9/015

(54) **Verfahren zur Desodorierung und Entkeimung von Gasen**
Method for deodorization and sterilisation of gases
Procédé pour la désodorisation et la stérilisation des gaz

(30) Priorität: 26.08.1994 DE 4430209
(43) Veröffentlichungstag der Anmeldung: 28.02.1996
(73) Patentinhaber: Ticona GmbH, 65451 Kelsterbach (DE); R&M Kühllagerbau GmbH, 80992 München (DE)
(72) Erfinder: von Eysmondt, Jörg, Dr., D-65719 Hofheim (DE); Schleicher, Andreas, Dr., D-65615 Beselich (DE); Schweers, Elke, Dr., D-69812 Bad Soden (DE); Fricke, Martin, D-99091 Erfurt (DE)

(56) Entgegenhaltungen:
- EP-A- 0 297 744
- DE-A- 3 905 842
- DE-A- 4 314 734
- US-A- 4 990 311

## Beschreibung

Die Erfindung betrifft ein Verfahren, bei dem Gase, die mit Mikroorganismen oder Geruchsstoffen beladen sind, durch Ozon und UV-Strahlung gleichzeitig desodoriert und entkeimt und dann mit Hilfe von Ozon-bindenden Polymeren von Rest-Ozon befreit werden.

Es ist bekannt, daß Luft mit Hilfe von Ozon desodoriert werden kann (M.J. Kirchner in Ullmanns Encyclopedia of Industr. Chemistry, Vol. A, W. Gerhartz, B. Elvers, VCH Verlagsgesellschaft mbH, Weinheim 1991, S. 355). Relevante geruchsbildende Stoffe sind z.B. Aromaten, Ammoniak, schwefelhaltige Verbindungen wie Schwefelwasserstoff, Methylsulfid und Methyldisulfid, Mercaptane, Amine, Acetaldehyd oder Ethylen. Sie liegen im allgemeinen in niedrigen Konzentrationen vor. Diese werden durch Ozon zu geruchslosen und in der Regel harmlosen Verbindungen oxidiert. Zur Ozon-Erzeugung werden üblicherweise Hochspannungs-Generatoren eingesetzt, die über eine stille elektrische Entladung Ozon aus Sauerstoff bilden. Nachteilig dabei ist der hohe apparative Aufwand der Ozon-Erzeugung (Sauerstoffversorgung, Gastrocknung, Hochspannungstechnik).

Methoden zur Rest-Ozon-Vernichtung werden vielfältig beschrieben.

So ist aus JP-A-06 063 356 die Verwendung eines Ozon zersetzenden Katalysators auf der Basis von SiO₂, Al₂O₃ und/oder TiO₂ auf einem Aktivkohle-Trägermaterial bekannt. Die Standzeiten dieser Katalysatoren sind jedoch in der Regel kurz, insbesondere bei komplex zusammengesetzten Geruchsstoffen, die zumeist adsorptiv von der Kohle gebunden werden.

In JP-A-04 138 167 wird ein Ozon zersetzender Katalysator beschrieben, der Mangan- und Kupferoxide enthält. Diese Schwermetallverbindungen haben den Nachteil, daß die Entsorgung dieser Materialien nach Gebrauch Probleme bereitet und daß durch unerwünschte Nebenreaktionen mit Schwefelwasserstoff und Merkaptanen ebenfalls eine rasche Desaktivierung eintreten kann. Dieser Effekt wird auch bei allen Edelmetall-Katalysatoren beobachtet.

Die Anwendung von UV-Strahlung der Wellenlänge 254 nm ermöglicht ebenfalls eine Ozon-Zerstörung, da Ozon bei dieser Wellenlänge intensiv UV-Licht absorbiert. So wird in JP-A-04 035 667 UV-Licht zur Zerstörung von restlichem Ozon nach Desodorierung der Luft genannt. Die UV-Strahlung erlaubt jedoch nur bei hohem Energieaufwand bzw. hoher Strahlungsdichte eine hinreichende Ozon-Zerstörung auf Restgehalte unter 0,1 ppm Ozon (MAK-Wert).

In EP-A-0 560 690 wird ein Gasreinigung-System beschrieben, bei dem die Erzeugung von UV-Strahlung und Ozon in zwei unabhängigen Generatoren erfolgt, die nach unterschiedlichen physikalischen Prinzipien arbeiten. Der apparative Aufwand ist hierbei relativ hoch. Über die Zerstörung von Ozon wird nichts ausgesagt.

Die Aufgabe der Erfindung war es daher, die bestehenden Nachteile zu vermeiden beziehungsweise ein einfacheres Verfahren zu entwickeln.

Die Erfindung betrifft ein Verfahren zur Desodorierung und Entkeimung von Gasen mit Ozon, dadurch gekennzeichnet, daß das mit Ozon behandelte Gas zur Beseitigung von Rest-Ozon mit einem Filtermaterial in Kontakt gebracht wird, das mindestens ein Ozon-bindendes Polymer enthält.

### Ozon-bindende Polymere

Ozon-bindende Polymere sind beispielsweise schwefelhaltige Polymere, Polyarylenether oder polymere Adsorbentien.

Polyarylenether bezeichnen Polymere, die Methyl-substituierte Arylenether-Einheiten enthalten. Als Polyarylenether wird 2,6-Dimethyl-Polyphenylenoxid bzw. 2,6-Dimethyl-Polyphenylenether (zu Eigenschaften und Herstellung siehe Ullmann's Encyclopedia of Industrial Chemistry, Vol. A21, 5. Auflage, B. Elvers, S. Hawkins und G. Schulz (Eds.), VCH Publishers, Weinheim-New York 1992, S. 605 ff.) bevorzugt verwendet.

Der Ausdruck schwefelhaltige Polymere umfaßt Polymere, die mindestens eine Arylenthioether-Einheit (-Ar-S-; Ar: Arylen) oder eine aliphatische Thioether-Einheit (-Alk-S-; Alk: Alkylen) enthalten, z.B. Polyarylenthioether oder Polysulfide. Die Arylengruppen können aus ein- oder mehrkernigen Aromaten bestehen. Die Arylengruppen bestehen aus mindestens einem fünf- oder sechsgliedrigen Ring, der ein oder mehrere Heteroatome enthalten und gegebenenfalls substituiert sein kann. Heteroatome sind z.B. Stickstoff oder Sauerstoff, Substituenten sind z.B. lineare oder verzweigte Alkylgruppen. Die schwefelhaltigen Polymere können außer Schwefelbrücken (-S-) auch Sulfoxidgruppen (-SO-) oder Sulfongruppen (-SO₂-) enthalten. Arylene sind beispielsweise Phenylen, Biphenylen (-C₆H₄-C₆H₄-) oder Naphthylen, die ein-oder mehrfach substituiert sein können. Substituenten sind z.B. geradkettige, cyclische oder verzweigte C₁-C₂₀-Kohlenwasserstoffreste, wie C₁-C₁₀-Alkylreste, z.B. Methyl, Ethyl, n-Propyl, iso-Propyl, n-Butyl, t-Butyl oder n-Hexyl, oder C₆-C₁₄-Arylreste, z.B. Phenyl oder Naphtyl; Halogenid-, Sulfonsäure-, Amino-, Nitro-, Cyano-, Hydroxy-, oder Carboxylgruppen.

Die Synthese von sulfongruppenhaltigen Polyarylenthioethern ist in Chimia 28(9), (1974) 567 beschrieben.

Polyphenylensulfid (abgekürzt PPS; Eigenschaften und Herstellung siehe "Ullmann's Encyclopedia of Industrial Chemistry", Volume A21, B. Elvers, S. Hawkins und G. Schulz (Eds.), VCH, Weinheim-New York 1992, S. 463 ff) wird als schwefelhaltiges Polymer bevorzugt verwendet. Die Arylengruppen im PPS können 1,4- und/oder 1,3-Verknüpfungen aufweisen. PPS bezeichnet sowohl das lineare als auch das vernetzte Polymer. Ferner kann PPS pro Arylengruppe unabhängig voneinander 1 bis 4 funktionelle Gruppen enthalten, z.B. Alkylreste, Halogene, Sulfonsäure-, Hydroxy-, Amino-, Nitro-, Cyano- oder Carboxygruppen.

Polymere Adsorbentien sind Polymere mit einer besonders großen Oberfläche. Solche Polymere sind zum Beispiel sogenannte Adsorberharze, die in der Technik verbreiteten Einsatz finden. Als Adsorberharze werden makroporöse oder makroretikulare Copolymere auf Basis Styrol/Divinylbenzol mit variierendem Vernetzungsgrad eingesetzt. Adsorberharze können auch aus Copolymeren auf Basis von Methacrylat/Ethylenglykol bestehen.

Polymere Adsorbentien können beispielsweise aus Copolymeren bestehen oder Copolymere enthalten, die wiederkehrende Struktureinheiten der Formel I 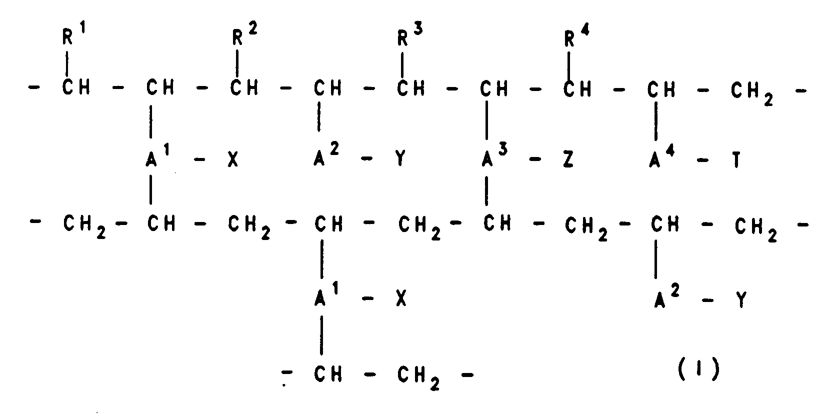 oder der Formel II 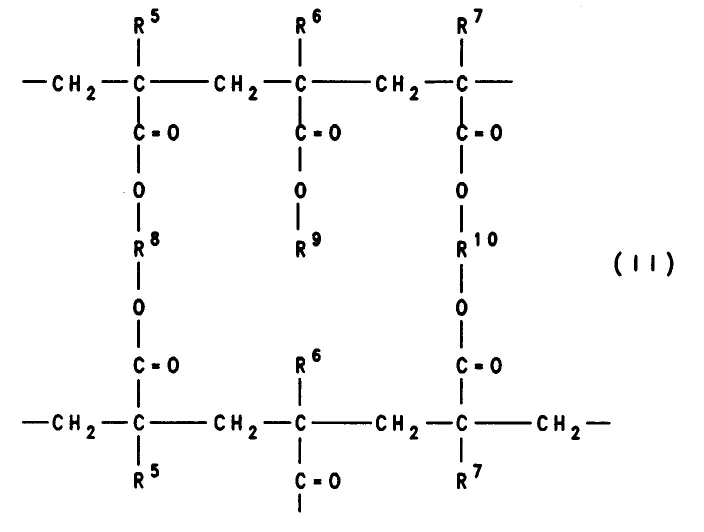 enthalten, worin A¹, A², A³, A⁴, X, Y, Z und T gleich oder verschieden sind und substituierte oder unsubstituierte Arylensysteme mit insgesamt 6 bis 18 C-Atomen bedeuten, R¹ bis R⁷ und R⁹ gleich oder verschieden sind und ein Wasserstoffatom oder eine Alkylgruppe mit 1 bis 20 C-Atomen, eine Hydroxygruppe oder ein Halogenatom bedeuten und R⁸ und R¹⁰ unabhängig voneinander eine C₂ - C₁₀-Alkylengruppe sind.

### Angaben zum Verfahren

Das zu behandelnde Gas ist vorzugsweise ein sauerstoffhaltiges Gas und kann beispielsweise Luft sein.

Das Ozon kann besonders einfach und wirtschaftlich durch UV-Bestrahlung erzeugt werden, wenn das zu behandelnde Gas Sauerstoff enthält. Einem Gas kann auch Sauerstoff zugesetzt werden. Ozon kann dann erzeugt werden, indem beispielsweise das zu behandelnde Gas, z.B. Luft, mit einem Niederdruck-UV-Strahler mit ozonbildendem Strahlungsspektrum bestrahlt wird.

Die Kontaktzeit beziehungsweise die Reaktionszeit des zu behandelnden Gases mit dem Ozon kann dadurch verlängert werden, daß das Ozon-haltige Gas durch eine Verweilzeitstrecke geleitet wird oder bei diskontinuierlicher Verfahrensweise einfach in einem Teil der Apparatur, das beispielsweise durch Ventile oder Klappen von der übrigen Apparatur abgeschlossen werden kann, stehen läßt.

Besonders vorteilhaft erweist es sich, das Gas vor der Ozon-Behandlung oder nach der Rest-Ozon-Beseitigung durch ein Partikel- und/oder ein Aerosolfilter zu leiten. Das Partikelfilter sollte zur Entfernung von Keimen aus dem Gas geeignet sein.

Das Verfahren gemäß der Erfindung hat die Vorteile, daß
- Luft gleichzeitig entkeimt und Gerüche entfernt werden,
- es unempfindlich gegenüber flüchtigen Schadstoffen ist und damit lange Standzeiten aufweist
- es apparativ einfach und kostengünstig ist.

Das Verfahren gemäß der Erfindung kann zum Beispiel in folgender Weise durchgeführt werden, wobei Luft desodoriert und entkeimt wird. Zunächst wird das zu behandelnde Gas über einen oder mehrere Vorfilter zur Partikel- und Aerosolabscheidung geleitet. Diese Vorfilter sind bekannt und handelsüblich. Anschließend wird der Gasstrom an einer Niederdruck-UV-Lampe (z.B. 15 Watt-Lampe), wie sie in der EP-A-0602505 beschrieben ist, vorbeigeleitet. Diese Vorrichtung emittiert die Strahlung als Linienstrahler im Wellenlängenbereich von 180 bis 260 nm, vorzugsweise im Bereich von 184 nm bis 253,7 nm (UV-C-Bereich), wobei gleichzeitig Ozon gebildet wird. Anschließend erfolgt die Reaktion von Ozon mit Geruchsstoffen und Keimen des Gases in einer Verweilzeitstrecke, die ein durchströmtes Rohr, ein Lüftungsschacht, ein Behälter oder ein kompletter Raum sein kann. Die Verweilzeit des mit Ozon angereicherten Gases im Behälter bzw. im Raum kann beliebig eingestellt werden. Nach erfolgter Entkeimung und Desodorierung wird das Gas über eine Adsorptionsvorrichtung zur Beseitigung von Rest-Ozon geleitet. Die Adsorptionsvorrichtung enthält ein oder mehrere Ozon-bindende Polymere.

Die simultane Entkeimung durch UV-Licht und Ozon in einem Verfahrensschritt ermöglicht eine wirksamere und schnellere Luttentkeimung als konventionelle UV-Strahler, die kein Ozon bilden. Enthält die Luft neben Mikroorganismen (Keimen) noch leichtflüchtige Geruchsstoffe, so werden diese ebenfalls sehr effektiv durch Ozon zerstört.

Die Rest-Ozonvernichtung mittels Ozon-bindender Polymere bringt folgende Vorteile:
- die Verminderung des Ozongehalts ist sehr effektiv (kleiner 0,001 ppm Rest-Ozongehalt)
- es erfolgt keine Vergiftung des Materials durch flüchtige Luftschadstoffe (z.B. CH₃-SH, H₂S, NH₃, SO₂).
- die Ozon-bindenden Polymere arbeiten auch bei niedrigen Temperaturen und hoher Luftfeuchtigkeit zuverlässig.

Diese Arbeitsweise weist gegenüber herkömmlichen Verfahren folgende Vorteile auf:
- Durch einen üblichen Vorfilter werden Staubpartikel und Aerosole entfernt, die sich auf dem UV-Strahler niederschlagen können, und damit die Emissionsleistung vermindern. Bakterien, Viren, Pilzsporen und geruchsbildende Substanzen passieren den Filter jedoch ungehindert und gelangen in die Oxidationskammer.
- Während konventionelle Quecksilber-Hochdruckstrahler durch Emission der biozid wirkenden Strahlung der Wellenlänge von 253,7 nm hauptsächlich entkeimend wirken, aber nur mäßig oxidierend, erlaubt der Niederdruck-UV-Strahler durch Verwendung geeigneter Spezial-Quarze mit hoher Transmission eine strahleninduzierte Ozon-Bildung durch die UV-Strahlung von 184 nm Wellenlänge. Hierdurch ist eine simultane Entkeimung und Desodorierung der Luft in einem Verfahrensschritt möglich.
- Ozon und UV-Strahlung ergänzen sich in ihrer keimtötenden Wirkung, so daß das Verfahren eine besonders breite und schnelle mikrobizide Wirksamkeit zeigt. Während Ozon durch seine starke Oxidationskraft entkeimend wirkt, erfolgt durch UV-C-Strahlung im wesentlichen eine photochemische Abtötung der Mikroorganismen.
- Die energetische Ausnutzung des Niederdruck-UV-Strahlers ist besser als bei konventionellen UV-Lampen, da nur wenig Energie in Form von Wärme oder sichtbarem Licht abgegeben wird. Ein hoher Anteil der elektrischen Energiemenge wird in UV-Strahlungsenergie (ca. 40 %) oder chemisch gebundene Energie umgewandelt (ca. 10 bis 20 % für Ozonbildung).
- Die Ozon-Erzeugung durch die beschriebenen Niederdruck-UV-Strahler kann direkt aus Umgebungsluft erfolgen, ohne daß eine vorherige Lufttrocknung notwendig wäre. Die Bildung von störenden Stickoxiden (NOₓ) ist wesentlich geringer als bei der Ozon-Erzeugung über stille elektrische Entladung. Aufgrund der störenden Stickoxidbildung müssen Hochspannungs-Ozon-Generatoren meist mit reinem Sauerstoff betrieben werden, oder es ist eine aufwendige Luftrocknung erforderlich, um die Bildung von korrosiver Salpetersäure aus NO₂ zu vermeiden.
- Für die Entkeimung und Geruchseliminierung werden im allgemeinen relativ niedrige Ozon-Konzentrationen im Bereich von 10 bis 2000 ppm, vorzugsweise 100 bis 1000 ppm benötigt. Dieses ist genau der Konzentrationsbereich, der mit der beschriebenen UV-Technik erzeugt werden kann. Lediglich bei höheren Ozon-Konzentrationen (> 10000 mg/m³) zeigt die Ozon-Herstellung über stille Entladung im Hochspannungsfeld wirtschaftliche Vorteile, wenn allein die Ozon-Erzeugung betrachtet wird.
- die Rest-Ozonzerstörung durch die organischen Polymere ist sehr effektiv (kleiner 0,001 ppm Rest-Ozon-Gehalt). Insbesondere Polyphenylensulfid zeichnet sich durch folgende Eigenschaften aus:
   - Ozon-Zersetzung im Temperaturbereich von minus 50 bis 150°C, vorzugsweise minus 10 bis 80°C,
   - unempfindlich gegen Luftfeuchte und Spurengase (z.B. CH₃-SH) oder flüchtige Anorganika (z.B. H₂S, NH₃, SO₂),
   - keine Adsorption von Kohlenwasserstoffen und den meisten anderen Lösemitteln
   - schwer entflammbar, hoher Schmelzpunkt (> 300°C),
   - ungiftig.

Durch das Verfahren gemäß der Erfindung werden bei der Entkeimung in dem Gasstrom enthaltende Mikroorganismen und Viren aller Art abgetötet, insbesondere Bakterien, Pilze sowie Pilzsporen.

Die Ozon-bindenden Polymere können als Pulver, Granulat, Faser, Vlies, Filz, Gewebe, Folie, gesintertes Material, Schaum, Formkörper oder als Beschichtung oder Imprägnierung von Trägermaterialien eingesetzt werden. Insbesondere sind Formkörper mit besonders großer Oberfläche, z.B. mit Gitter- oder Wabenstruktur, geeignet. Die Pulver besitzen z.B. handelsübliche Teilchengrößen, wobei auch Granulate verwendbar sind. Wichtig hierbei ist es, daß das zu behandelnde Gas oder die Flüssigkeit durch das Polymermaterial, beispielsweise in Form eines Pulver-Festbettes, ohne Störung durchgeleitet werden kann. Werden die Polymere als Fasern verwendet, können diese als Stapelfasern, Nadelfilz, "non woven" Material, Kardenband oder Gewebe eingesetzt werden. Auch Folien oder Folienschnipsel können in geeigneter Form Verwendung finden.

Die Ozon-bindenden Polymere sollten in einer Form mit möglichst großer Oberfläche vorliegen um eine optimale Filterwirkung entfalten zu können. Beschichtungen von Trägermaterialien mit Ozon-bindendem Polymer wie Polyphenylensulfid oder 2,6-Dimethyl-Polyphenylenoxid können durch Auftragen von Lösungen des schwefelhaltigen Polymers auf das Trägermaterial erhalten werden. Imprägnierungen werden z.B. durch Tränken eines saugfähigen Trägermaterials hergestellt. Als Trägermaterial werden im allgemeinen anorganische Stoffe wie Glas, Kieselgel, Aluminiumoxid, Sand, keramische Massen, Metall und organische Stoffe wie Kunststoffe eingesetzt.

Auf die Ozon-bindenden Polymere können auch z.B. Metalle, insbesondere Edelmetalle und Übergangsmetalle, oder Metalloxide wie Übergangsmetalloxide, beispielsweise durch Aufimprägnieren aufgebracht werden, die dann z.B. in Form kleiner Cluster vorliegen.

Das Ozon-bindende Polymer kann im allgemeinen als unverschnittenes Material eingesetzt werden. Möglich ist aber auch der Zusatz von üblichen Füllstoffen, wie Kreide, Talk, Ton, Glimmer, und/oder faserförmigen Verstärkungsmitteln, wie Glas- und/oder Kohlenstoffasern, Whiskers, sowie weiteren üblichen Zusatzstoffen und Verarbeitungshilfmitteln, z.B. Gleitmittel, Trennmittel, Antioxidantien, UV-Stabilisatoren.

## Patentansprüche

1. Verfahren zur Desodorierung und Entkeimung von Gasen mit Ozon, dadurch gekennzeichnet, daß das mit Ozon behandelte Gas zur Beseitigung von Rest-Ozon mit einem Filtermaterial in Kontakt gebracht wird, das mindestens ein Ozon-bindendes Polymer enthält.

2. Verfahren nach Anspruch 1, dadurch gekennzeichnet, daß ein polymeres Adsorbens, ein Polyarylenthioether und/oder ein Polyarylenether als Ozonbindendes Polymer verwendet wird.

3. Verfahren nach Anspruch 1 oder 2, dadurch gekennzeichnet, daß zur Ozon-Erzeugung ein Niederdruck-UV-Strahler verwendet wird.

4. Verfahren nach den Ansprüchen 1 bis 3 dadurch gekennzeichnet, daß zur Ozon-Erzeugung ein Niederdruck-UV-Strahler verwendet wird, der Strahlung der Wellenlänge 184 und/oder 253,7 nm emittiert.

5. Verfahren nach einem oder mehreren der Ansprüche 1 bis 4, dadurch gekennzeichnet, daß bei Gastemperaturen im Bereich von minus 50 bis 150°C, vorzugsweise bei minus 10 bis 80°C gearbeitet wird.

6. Verfahren nach einem oder mehreren der Ansprüche 1 bis 5, dadurch gekennzeichnet, daß das zu behandelnde Gas Luft ist.

## Claims

1. A process for deodorizing and sterilizing gases using ozone, which comprises bringing the gas treated with ozone into contact, to eliminate residual ozone, with a filter material which comprises at least one ozone-binding polymer.

2. The process as claimed in claim 1, wherein a polymeric adsorbent, a polyarylene thioether and/or a polyarylene ether is used as the ozone-binding polymer.

3. The process as claimed in claim 1 or 2, wherein a low pressure UV lamp is used for generation of the ozone.

4. The process as claimed in claims 1 to 3, wherein a low pressure UV lamp which emits radiation of wavelength 184 and/or 253.7 nm is used for generation of the ozone.

5. The process as claimed in one or more of claims 1 to 4, which is carried out at gas temperatures in the range from minus 50 to 150°C, preferably at minus 10 to 80°C.

6. The process as claimed in one or more of claims 1 to 5, wherein the gas to be treated is air.

## Revendications

1. Procedé de désodorisation et de stérilisation de gaz avec de l'ozone, caractérisé en ce que le gaz traité avec de l'ozone est amené, afin d'éliminer l'ozone résiduel, au contact d'un matériau filtrant qui contient au moins un polymère se liant à l'ozone.

2. Procédé suivant la revendication 1, caractérisé en ce que l'on utilise un adsorbant polymère, un thioéther de polyarylène et/ou un éther de polyarylène en tant que polymère se liant à l'ozone.

3. Procédé suivant la revendication 1 ou 2, caractérisé en ce que l'on utilise une lampe UV à basse pression pour produire l'ozone.

4. Procédé suivant les revendications 1 à 3, caractérisé en ce que l'on utilise pour produire l'ozone une lampe UV à basse pression qui émet un rayonnement de longueur d'onde 184 et/ou 253,7 nm.

5. Procédé suivant une ou plusieurs des revendications 1 à 4, caractérisé en ce que l'on travaille à des températures de gaz dans l'intervalle de -50 à 150°C, de préférence de -10 a 80°C.

6. Procédé suivant une ou plusieurs des revendications 1 à 5, caractérisé en ce que le gaz à traiter est de l'air.
